# EUROPÄISCHE PATENTSCHRIFT

(11) **EP 0 839 010 B1**
(45) Veröffentlichungstag und Bekanntmachung des Hinweises auf die Patenterteilung: **26.03.2003**
(21) Anmeldenummer: 96926340.9
(22) Anmeldetag: 12.07.1996
(51) Int. Cl.: A45D 29/00

(54) **VERFAHREN ZUM BEHANDELN VON NATÜRLICHEN FINGERNÄGELN ODER VON FUSSNÄGELN**
METHOD FOR THE TREATMENT OF NATURAL FINGERNAILS OR TOENAILS
PROCEDE PERMETTANT DE TRAITER DES ONGLES DE MAIN NATURELS OU DES ONGLES DE PIED

(30) Priorität: 15.07.1995 DE 29511466 U; 02.02.1996 DE 19603716
(43) Veröffentlichungstag der Anmeldung: 06.05.1998
(73) Patentinhaber: Homann, Steffen, 04746 Hartha (DE)
(72) Erfinder: Homann, Steffen, 04746 Hartha (DE)
(74) Vertreter: Goy, Wolfgang, Dipl.-Phys.
(86) Internationale Anmeldenummer: EP9603071
(87) Internationale Veröffentlichungsnummer: WO97003585

(56) Entgegenhaltungen:
- EP-A- 0 031 147
- EP-A- 0 170 482
- DE-A- 1 557 314
- FR-A- 2 212 746
- US-A- 2 569 250
- PATENT ABSTRACTS OF JAPAN vol. 94, no. 010 & JP,A,06 292683 (TSUNEYUKI ITANO), 21.Oktober 1994,

## Beschreibung

Die Erfindung betrifft ein Verfahren zum Behandeln des vorderen Nagelrandes von Fingernägeln oder Fußnägeln.

Das erfindungsgemäße Verfahren sowie die dazugehörige Vorrichtung zum Behandeln von Finger- und Fußnägeln findet ihr Einsatzgebiet im kosmetischen Bereich für Maniküre sowie Pediküre. Die bisherige Behandlung von Finger- und Fußnägeln sieht so aus, daß diese lediglich geschnitten und bei Bedarf anschließend noch gefeilt werden. Nachteil ist aber oft, daß die Nägel brüchig sind und einreißen mit den damit verbundenen Nagelproblemen. Die Folge davon ist, daß aufgrund des zackigen vorderen Nagelrandes man oft an Stoffen hängenbleibt und im Extremfall sogar Fäden zieht. Auch sehen rissige und brüchige Nägel ungepflegt aus.

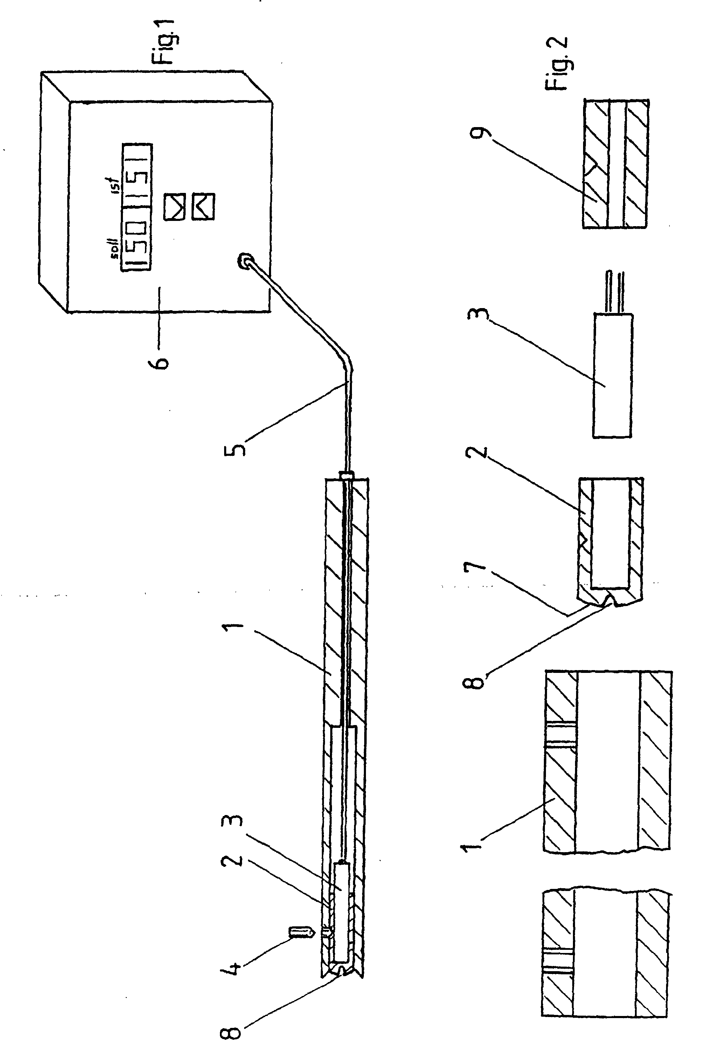

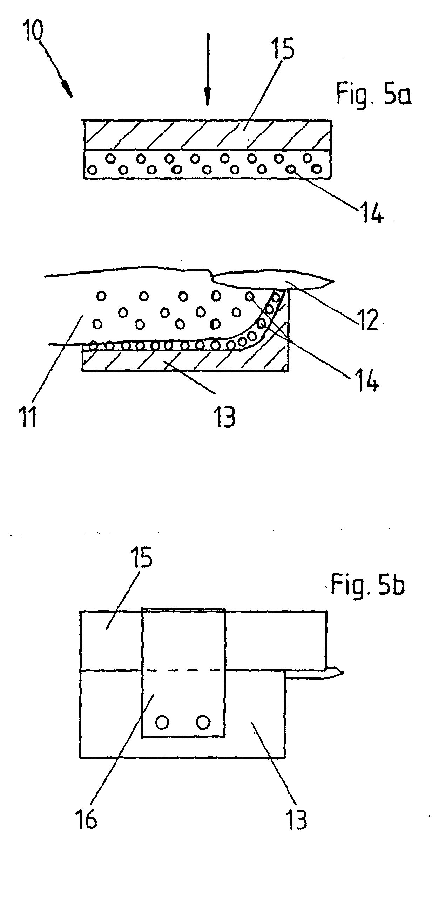

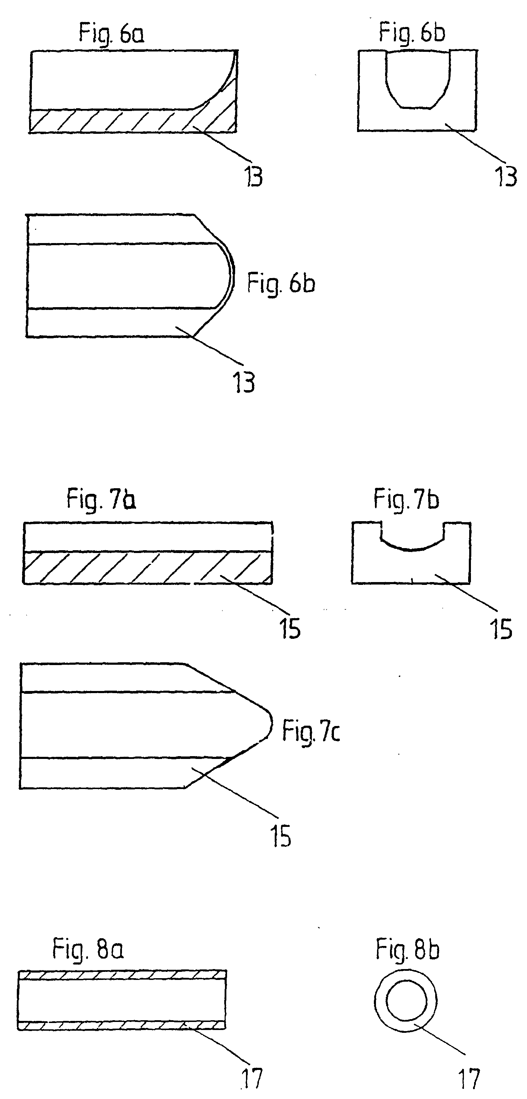

In der EP-A-0 170 482 sind zwei Vorrichtungen zum Behandeln von Nägeln offenbart. - Bei der ersten Ausführungsform der Fig. 1 bis 4 ist eine Zange zum Behandeln von eingewachsenen Nägeln offenbart. Der eine Schenkel der Zange weist einen längsverlaufenden, elektrisch beheizbaren Draht auf. Dazu korrespondierend weist der andere Schenkel einen Schlitz zur Aufnahme des Drahtes auf. Das Grundprinzip dieser Zange besteht darin, daß mit den beiden Schenkeln der eingewachsene Nagel zunächst gegriffen wird. Anschließend wird der Draht elektrisch beheizt, um so den zu behandelnden Nagel lokal zu erhitzen. Der Nagel erweicht dabei und kann in eine bessere Form zurückgebogen werden. Anschließend wird der Nagel gekühlt. - Bei der zweiten Ausführungsform der Fig. 5 und 6 ist eine Vorrichtung zum Behandeln von eingerollten Nägeln offenbart. Das Instrument weist dabei einen zylinderförmigen Grundkörper auf, welcher auf seiner Oberseite mit einem elektrischen Widerstandsheizdraht versehen ist. Über dem zylinderförmigen Körper ist eine gewölbte Metallplatte von Bolzen gehalten. Dabei drücken Federn diese gewölbte Metallplatte von dem zylinderförmigen Grundkörper weg. Um einen eingerollten Nagel zu behandeln, wird dieser in den Zwischenraum zwischen der mit dem Heizdraht versehenen Oberfläche des zylinderförmigen Körpers und der Metallplatte eingeschoben. Die gewölbte Abdeckplatte wird anschließend nach unten gedrückt, so daß der Nagel fest zwischen dem Heizdraht und der gewölbten Abdeckplatte zu liegen kommt. Anschließend wird die Heizeinrichtung in Gang gesetzt und der Nagel auf eine derartige Temperatur erhitzt, daß er erweicht und in eine bessere Form zurückgebogen werden kann.

Der Erfindung liegt die **Aufgabe** zugrunde, ein Verfahren zum Behandeln des vorderen Nagelrandes von Fingernägeln oder Fußnägeln zu schaffen.

Die technische **Lösung** ist gekennzeichnet durch die Merkmale im Kennzeichen des Anspruchs 1.

Die Grundidee und der Vorteil des erfindungsgemäßen Behandlungsverfahrens liegt darin, daß die Hornsubstanz am vorderen Nagelende durch die Erwärmung leicht schmilzt und verschweißt und somit versiegelt wird. Der Nagel kann somit nicht mehr einreißen und wird auch nicht mehr brüchig. Durch das Verschmelzen der Hornsubstanz ergibt sich darüber hinaus eine glatte Fläche am vorderen Nagelrand, so daß kein Angriffspunkt zum Hängenbleiben oder dgl. an einem Stoff besteht. Schließlich wird der vordere Nagelrand verstärkt und stabilisiert. Indem die Behandlungseinrichtung am vorderen Nagelrand entlanggeführt wird, kann sie jeder gewünschten Nagelkontur folgen.

Die Weiterbildung gemäß Anspruch 2 stellt die technisch einfachste Möglichkeit zur Schaffung einer beheizbaren Einrichtung dar, welche am vorderen Nagelrand entlangführbar ist. Vorzugsweise wird dabei die gekrümmte Mantelfläche dazu verwendet, um die beheizbare Einrichtung am vorderen Nagelrand entlangzuführen. Selbstverständlich kann aber auch die Stirnfläche des Zylinders verwendet werden, wobei diese selbstverständlich profiliert sein kann. So ist es denkbar, außer einer ebenen Stirnfläche auch eine konvex oder konkav gekrümmte Stirnfläche zu verwenden. Auch andere gekrümmte Profilformen sind denkbar, mit denen ein Entlangführen am vorderen Nagelrand möglich ist.

Grundsätzlich ist es denkbar, daß die Behandlungsvorrichtung in der Art einer Feile oder Schere ausgebildet ist. Eine bevorzugte Ausbildungsform schlägt jedoch Anspruch 3 vor. Die schlitzartige Nut zur Aufnahme des vorderen Nagelendes dient dabei gewissermaßen als Führungsnut, welche den Nagel auch teilweise umgreift, so daß nicht nur das vordere, stimseitige Ende des Nagels versiegelt wird, sondem auch die benachbarten Ober- und Unterseiten des Nagels. Darüber hinaus läßt sich die Behandlungseinrichtung durch diese Führungsnut einfacher handhaben.

Eine Weiterbildung hiervon schlägt Anspruch 4 vor. Insbesondere durch eine gekrümmte Nut ist eine optimale Versiegelung des vorderen Nagelrandes möglich, da entsprechend der Kontur dieses Nagelrandes die schlitzartige Nut optimal dem Konturenverlauf entlanggeführt werden kann. Je nach der Krümmungsform können beispielsweise Grob- und Feinarbeiten durchgeführt werden.

Für den Fall, daß die beheizbare Einrichtung zylinderförmig ausgebildet ist, wird in einer Weiterbildung der Nut gemäß Anspruch 5 vorgeschlagen, daß diese um den Umfang der zylinderförmigen Mantelfläche herum ausgebildet ist. Dies stellt eine Weiterbildung der einfachsten Variante dar, bei der lediglich die beheizbare Einrichtung durch einen Zylinder gebildet ist. Die Weiterbildung dieser einfachsten Variante sieht nunmehr vor, eine rundumgehende Nut zur Aufnahme des Nagels zu verwenden.

Eine erste Alternative der Wärmeversorgung für die Behandlungsvorrichtung schlägt Anspruch 6 vor. In der Praxis sieht dies dann so aus, daß die Behandlungsvorrichtung im Nichtgebrauchszustand sich im "Ofen" befindet, wo die beheizbare Einrichtung auf der gewünschten Betriebstemperatur gehalten wird. Für den Gebrauch wird dann die Behandlungsvorrichtung dem "Ofen" entnommen und kann dann für eine gewisse Zeit verwendet werden, solange die beheizbare Einrichtung die erforderliche Mindesttemperatur aufweist.

Der Vorteil der Alternativen gemäß Anspruch 7 besteht darin, daß immer die gewünschte Temperatur vorhanden und somit die Behandlungsvorrichtung immer optimal betriebsbereit ist. Hierzu ist allerdings ein elektrisches Steuer- und Regelgerät mit einem Stromzuführungskabel zu der Behandlungsvorrichtung notwendig. Das Aufbringen der Temperatur über die elektrische Widerstandsheizung erfolgt mit elektrischen Heizwiderständen, Heizfolien oder Heizpatronen. Die Heizfolien werden vorzugsweise auf einem Grundkörper aufgeklebt oder aufvulkanisiert. Weiterhin weist die elektrische Widerstandsheizung vorzugsweise einen Temperaturfühler auf, um eine entsprechende Temperaturregelung vornehmen zu können.

Eine Weiterbildung hiervon schlägt gemäß Anspruch 8 vor, daß die Heiztemperatur regelbar ist. Dies schließt ein, daß mittels des Steuergerätes die jeweils gewünschte Temperatur der Behandlungsvorrichtung eingestellt und vorgegeben werden kann, was von Fall zu Fall unterschiedlich sein kann. Um die Heiztemperatur zu regeln, ist vorzugsweise in der beheizbaren Einrichtung ein Thermofühler zur Kontrolle der anliegenden Temperatur vorgesehen.

Eine weitere Weiterbildung schlägt gemäß Anspruch 9 vor, daß die beheizbare Einrichtung ein in einem Halter angeordnetes Heizelement aufweist. Dies ist so zu verstehen, daß das Heizelement als separates Bauteil, insbesondere als Metalleinsatz in einem Handhabungsgehäuse angeordnet ist, welches stiftartig oder als griffähnlicher Stift ausgebildet sein kann. Der Vorteil eines derartigen Heizelementkörpers besteht darin, daß der Gesamtaufbau der Behandlungsvorrichtung technisch einfach ist, da lediglich das Heizelement in die Halterung eingesetzt werden muß. Insbesondere ist auch ein Austausch eines defekten Heizelements ohne weiteres möglich, ohne daß die Halterung dadurch unbrauchbar wird.

Durch die Weiterbildung gemäß Anspruch 10 kann auf handelsübliche Heizpatronen bzw. Heizkissen zurückgegriffen werden, welche dann jeweils in das speziell ausgebildete Heizelement eingesetzt werden. Zum einen ergibt sich dadurch eine konstruktive Ver-einfachung, zum anderen läßt sich die Behandlungsvorrichtung kostengünstig herstellen.

Die Zusatzeinrichtung gemäß Anspruch 11 hat die Funktion eines Hilfsmittels zum verbesserten Arbeiten am Nagel. Die Grundidee liegt in einem Fingerschutzhalter, welcher den gesamten Finger umschließt und nur die Nagelspitze freiläßt. Dieser Fingerschutzhalter ist mit feuchter, dünner Watte oder einem vergleichbaren Material wie Leinen, Veloursleder etc. ausgelegt. Diese feuchte Watte dient der Kühlung. Selbstverständlich ist es auch denkbar, die Watte mit einem Pflegemittel zu versehen. Auch ist es möglich, eine äußere Halterung vorzusehen, welche ein innenliegendes und dieses umschließendes Kissen beispielsweise aus Silicon oder Kunststoff aufweist. Durch dieses Kissen kann mittels einer Pumpe eine Kühlflüssigkeit, beispielsweise Kühlwasser zwecks Kühlung des Fingers oder des Zehens hindurchgepumpt werden. Auch können die Kissen permanent mit einen Kühlmittel gefüllt und in sich geschlossen sein.

Die angelegte elektrische Spannung gemäß Anspruch 12 erzeugt eine elektrische Reizung.

Verschiedene Ausführungsbeispiele einer erfindungsgemäßen Vorrichtung zum Behandeln von Finger- und Fußnägeln wird nachfolgend anhand der Zeichnungen beschrieben. In diesen zeigt:
- Fig. 1: eine schematische Schnittansicht einer ersten Ausführungsform einer Vorrichtung zum Behandeln des vorderen Nagelrandes von Finger- und Fußnägeln;
- Fig. 2: eine hierzu etwas modifizierte Ausführungsform in einer Explosionsdarstellung;
- Fig. 3 a und b: eine erste Ausführungsform eines Heizelements mit einer konvexen Stimfläche in verschiedenen Ansichten;
- Fig. 4 a bis c: eine zweite Ausführungsform des Heizelements mit einer konkaven Stirnfläche in verschiedenen Ansichten;
- Fig. 5 a und b: einen Fingerschutzhalter für den zu behandelnden Finger;
- Fig. 6 a bis c: das Unterteil des Fingerschutzhalters in den Fig. 5 a und b;
- Fig. 7 a bis c: das Oberteil des Fingerschutzhalters in Fig. 5 a und b;
- Fig. 8 a und b: ein mit Kühlflüssigkeit gefülltes Kissen für den Fingerschutzhalter in Fig. 5 a und b;
- Fig. 9 a und b: eine dritte Ausführungsform der Behandlungsvorrichtung, bei der die beheizbare Einrichtung zylinderförmig ausgebildet ist und eine ringförmig umlaufende Nut aufweist;
- Fig. 10: eine vierte Ausführungsform entsprechend der dritten Ausführungsform, jedoch ohne Nut;
- Fig. 11 a bis c: eine fünfte Ausführungsform zum Polieren der Nageloberfläche sowie zum Entfernen und Zurückschieben der Nagelhaut;
- Fig. 12 a bis d: eine schematische Schnittansicht einer letzten Ausführungsform einer Vorrichtung zum Behandeln des vorderen Nagelrandes von Finger- und Fußnägeln in einer Explosionsdarstellung.

Fig. 1 zeigt eine erste Version einer Vorrichtung zum Behandeln von Finger- und Fußnägeln. Dabei ist ein stiftartiger sowie rohrFörmiger Halter 1 vorgesehen. In seiner stirnseitigen Öffnung befindet sich ein zylinderförmiges Heizelement 2. Dieses ist mit einer Art Sacklochbohrung versehen, in der eine Heizpatrone 3 angeordnet ist. Gehalten wird das Heizelement 2 im Halter 1 durch eine Madenschraube 4. Die handelsübliche Heizpatrone 3 weist rückseitig ein Kabel 5 auf, welches durch das hintere rohrförmig offene Ende des stabförmigen Halters 1 herausgeführt und an ein Regelgerät 6 angeschlossen ist, welches einerseits die Heizpatrone 3 mit Strom versorgt und dabei aufheizt und andererseits die Heiztemperatur auf einen konstanten Wert hält. Vor allem aber weist das Heizelement 2 an seiner vorderen Stirnseite 7 eine über den Durchmesser der Stimseite 7 sich erstreckende Nut 8 auf.

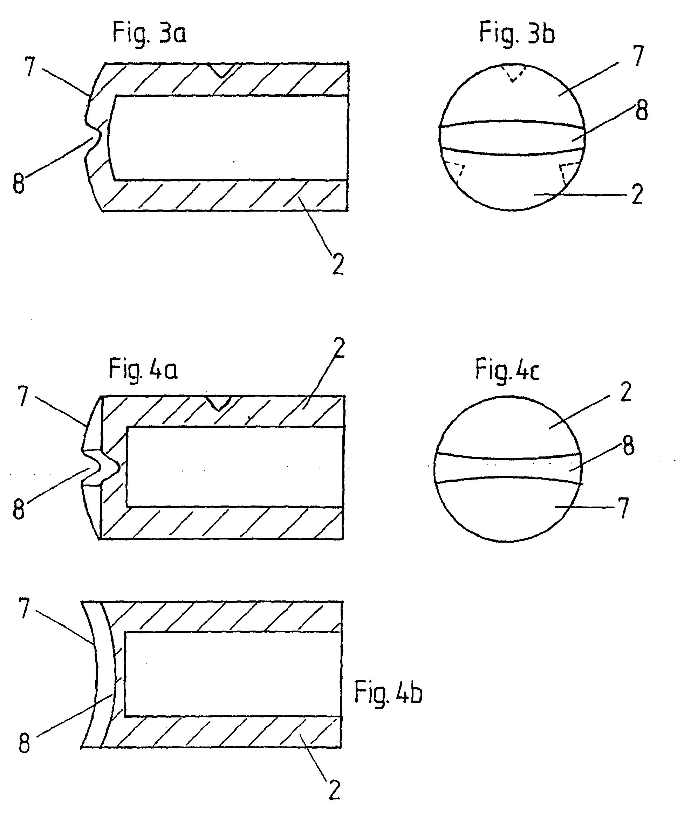

In den Fig. 3 a und b sowie Fig. 4 a bis c sind verschiedene Ausführungsformen der Heizelemente 2 mit unterschiedlich ausgebildeten Nuten 8 dargestellt. Bei der in Fig. 3 a und b dargestellten ersten Ausführungsform ist die Stimseite 7 des Heizelements 2 konvex gekrümmt, wie insbesondere die Schnittdarstellung in Fig. 3 a erkennen läßt. Fig. 3 b läßt erkennen, daß sich die schlitzartige Nut 8 über den gesamten Durchmesser des zylinderförmigen Heizelements 2 erstreckt. Das Heizelement 2 in der Ausführungsform der Fig. 4 a bis c ist dagegen mit seiner Stimseite 7 konkav ausgebildet, wie insbesondere die beiden Schnittdarstellungen in Fig. 4 a und b erkennen lassen, wobei es sich in Fig. 4 b ebenfalls um einen Längsschnitt, jedoch senkrecht zu dem in Fig. 4 a handelt. Auch hier ist in Fig. 4 c erkennbar, daß sich die schlitzartige Nut 8 über den gesamten Durchmesser der Stirnseite 7 erstreckt.

Die so ausgebildete Behandlungsvorrichtung für Finger- und Fußnägel funktioniert wie folgt:

Mittels des Regelgerätes 6 wird das Heizelement 2 auf eine ganz bestimmte, vorgegebene Temperatur aufgeheizt. Die behandelnde Person kann dann mittels des griffartigen Halters 1 entlang des vorderen Nagelrandes entlangfahren, indem dieser vordere Nagelrand von der Nut 8 aufgenommen wird. Durch die Hitzeeinwirkung auf den Nagel schmilzt etwas die Hornsubstanz und versiegelt somit den vorderen Nagelrand.

In Fig. 2 ist eine bezüglich Fig. 1 etwas modifizierte Ausführungsform der Behandlungsvorrichtung dargestellt, und zwar in einer Explosivdarstellung. Dadurch sollen die Einzelteile, nämlich der Halter 1, das Heizelement 2 sowie die Heizpatrone 3 besser erkennbar sein. Der einzige Unterschied zu der Ausführungsform in Fig. 1 besteht darin, daß die Ausführungsform in Fig. 2 statt des massiven hinteren rohrartigen Endes des Halters 1 ein separates Einsetzteil 9 aufweist, welches in den rohrförmigen Halter 1 eingeschoben und beispielsweise mittels einer Madenschraube fixiert wird.

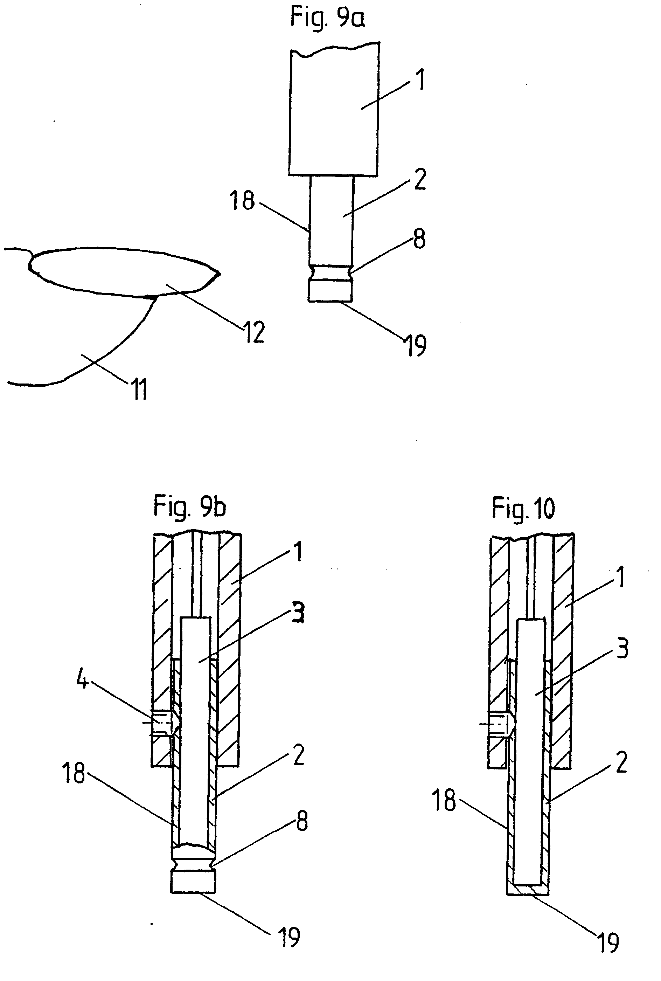

In Fig. 9 a und b ist in einer Ansicht sowie einer Schnittansicht eine dritte Ausführungsform der Behandlungsvorrichtung dargestellt. Auch hier ist wieder ein länglicher Halter 1 vorgesehen. In seiner stimseitigen Öffnung befindet sich ein zylinderförmiges Heizelement 2, in dem wiederum eine Heizpatrone 3 angeordnet ist. Wie in der Zeichnung erkennbar ist, ragt das vordere Ende des zylinderförmigen Heizelements 2 über die Stirnseite des Halters 1 hinaus. Außerdem weist das zylinderförmige Heizelement 2 im Bereich des vorderen Endes eine umlaufende Nut 8 auf. Diese Nut 8 nimmt dabei den vorderen Nagelrand eines Fingernagels 12 auf, wie insbesondere Fig. 9 a erkennen läßt.

In Fig. 10 ist eine vereinfachte Variante dieser dritten Ausführungsform dargestellt, wobei der einzige Unterschied darin besteht, daß diese vereinfachte Variante keine umlaufende Nut 8 aufweist, so daß der Benutzer entweder mit der Mantelfläche 18 oder der Stimfläche 19 am vorderen Nagelrand entlanggeführt wird, ohne daß dieser von einer Nut aufgenommen wird.

In Fig. 5 a und b ist ein Fingerschutzhalter 10 in einer schematischen Längsschnittdarstellung (Fig. 5 a) sowie in einer Seitenansicht in der Betriebsform des Fingerschutzhalters 10 (Fig. 5 b) dargestellt. Die Grundidee besteht dabei darin, den Finger 11 der zu behandelnden Person derart in diesen Fingerschutzhalter 10 gekühlt festzulegen, daß nur das vordere Ende des Fingernagels 12 herausschaut. Zu diesem Zweck weist der Fingerschutzhalter 10 ein wannenförmiges Unterteil 13 auf (Fig. 6 a bis c), in das der Finger 11 hineingelegt wird. Dabei ist der Finger 11 in der Wanne mit feuchter Watte 14 unterlegt, und der Fingernagel 12 ragt über die Oberkante des Unterteils 13 hinweg. Weiterhin weist der Fingerschutzhalter 10 ein Oberteil 15 auf, welches ebenfalls wannenförmig ausgebildet ist und die Oberseite des Fingers 11 abdeckt (Fig. 7 a bis c). In Fig. 5 a ist die Situation kurz vor dem Aufstecken des Oberteils 15 auf das Unterteil 13 dargestellt, wobei auch hier zwischen dem Oberteil 15 und dem Finger 11 feuchte Watte 14 angeordnet ist. In Fig. 5 b ist angedeutet, wie Unterteil 13 und Oberteil 15 mittels eines Gummibandes 16 (oder auch mittels eines Klettverschlusses) so gesichert sind, daß nur das vordere Ende des Fingemagels 12 aus dem Fingerschutzhalter 10 herausschaut. Durch die Kühlung mittels der feuchten Watte 14 wird verhindert, daß der Fingernagel 11 und die nicht behandelten Bereiche des Fingernagels 12 warm werden.

Statt der Watte 14 kann auch ein schlauchförmiges Kissen 17 aus Kunststoff oder Silicon verwendet werden, welches mit Kühlflüssigkeit gefüllt ist. Dieses Kissen 17 wird auf den Finger aufgestülpt und anschließend in den Fingerschutzhalter 10 hineingelegt. Auch hier tritt somit mittels der Kühlflüssigkeit eine Kühlung des Fingers 11 auf.

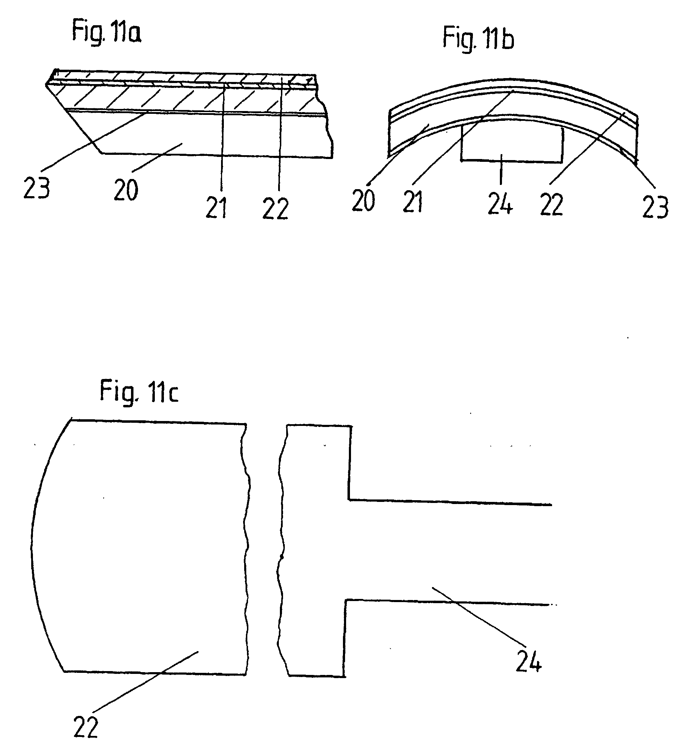

In den Fig. 11 a bis c ist eine Wärmebehandlungseinrichtung zum Polieren der Nageloberfläche und zum Entfernen und Zurückschieben der Nagelhaut dargestellt. Die Vorrichtung besteht dabei aus einem gewölbten Grundkörper 20. Dieser ist oberseitig mit einer aufgeklebten oder aufvulkanisierten Heizfolie 21 mit Temperaturfühler versehen. Diese Heizfolie 21 ist dabei sandwichartig zwischen dem gewölbten Grundkörper 20 und einer oberen Isolationsabdeckung 22 eingebettet. Unterseitig weist der gewölbte Grundkörper 20 eine Poliermittelbeschichtung 23 auf, bei der es sich um Polierpapier, um einen Polierstein oder ein vergleichbares Material handeln kann. Statt der Poliermittelbeschichtung 23 kann auch eine Polierpaste oder eine Poliercreme verwendet werden. Am rückseitigen Ende weist die Vorrichtung eine Befestigung 24 in einem nicht dargestellten Halter auf.

Zum Polieren der Nageloberfläche wird die Vorrichtung auf den Nagel aufgelegt und dabei hin- und herbewegt. Da die Unterseite des gewölbten Grundkörpers 20 erwärmt ist, schmilzt ein klein wenig die Oberfläche der Nagelsubstanz und vergleichmäßigt sie im Sinne eines Poliereffektes. Durch entsprechendes Bewegen der Vorrichtung kann zusätzlich noch die Nagelhaut entfernt und zurückgeschoben werden.

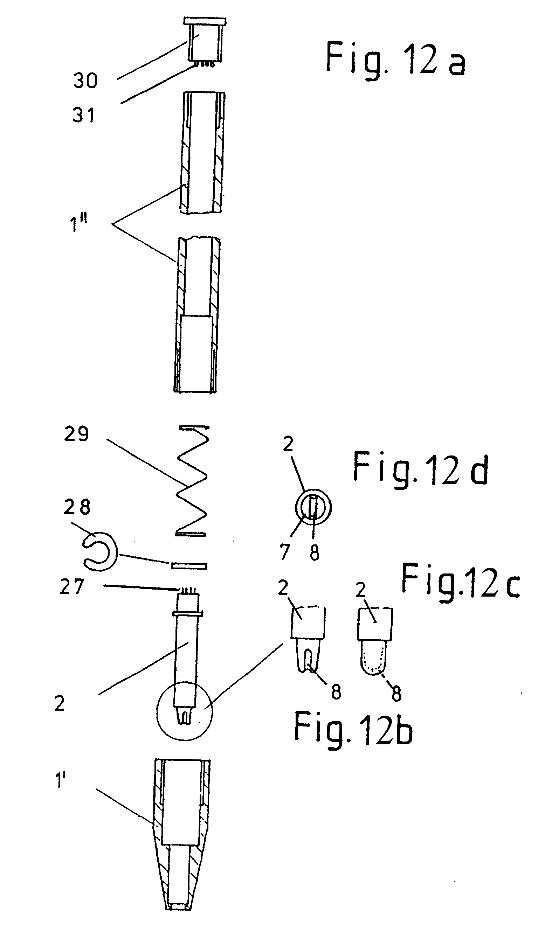

Schließlich ist in den Fig. 12 a bis d noch eine weitere Version einer Vorrichtung zum Behandeln von Finger- und Fußnägeln dargestellt, die vom Grundprinzip her derjenigen Vorrichtung entspricht, wie sie als erste Version in Fig. 1 dargestellt ist. Auch hier ist ein insgesamt rohrförmiger, stiftartiger Halter 1 vorgesehen, der aus einem hülsenförmigen Kopfteil 1' aus PTFE (Teflon) sowie dem eigentlichen Griffteil 1" zusammengesetzt ist. Das hülsenförmige Kopfteil 1' dient der Aufnahme eines patronenartigen Heizelements 2, indem dieses in das Kopfteil 1' eingesetzt wird. Wie in den einzelnen Darstellungen der Fig. 12 b bis d erkennbar ist, weist die Stirnseite 7 dieses Heizelements 2 eine Nut 8 auf. Rückseitig weist das patronenartige Heizelement 2 Anschlüsse 27 für eine elektrische Spannungsversorgung auf.

Im Montagezustand wird auf das in das Kopfteil 1' hineingesteckte Heizelement 2 eine Unterlegscheibe 28 aufgesteckt, sowie weiterhin eine wendelförmige Druckfeder 29. Schließlich wird das Griffteil 1" auf das Kopfteil 1' und die Fixierung des Heizelements 2 aufgeschraubt, wobei sich die Druckfeder 29 an einem entsprechenden Vorsprung im Innern des Griffteils 1" abstützt und das Heizelement 2 in die vordere Position innerhalb des Kopfteils 1' drückt. Das rückseitige Ende des Griffteils 1" ist durch eine sogenannte Dose durch Aufschrauben abgeschlossen. Diese Dose 30 weist ebenfalls Anschlüsse 31 in Form von Lötpins für die elektrischen Anschlüsse auf.

Zum Betrieb der Behandlungsvorrichtung wird über den elektrischen Anschluß das Heizelement 2 über eine entsprechende Widerstandsheizung aufgeheizt, so daß sich der Kopf des Heizelements 2 mit der Nut 8 erwärmt.

### Bezugszeichenliste

- 1: Halter
- 1': Kopfteil
- 1": Griffteil
- 2: Heizelement
- 3: Heizpatrone
- 4: Madenschraube
- 5: Kabel
- 6: Regelgerät
- 7: Stirnseite
- 8: Nut
- 9: Einsatzteil
- 10: Fingerschutzhalter
- 11: Finger
- 12: Fingernagel
- 13: Unterteil
- 14: Watte
- 15: Oberteil
- 16: Gummiband
- 17: Kissen
- 18: Mantelfläche
- 19: Stirnfläche
- 20: Grundkörper
- 21: Heizfolie
- 22: Isolationsabdeckung
- 23: Poliermittelbeschichtung
- 24: Befestigung
- 25: Halterung
- 26: Heizpatrone
- 27: Anschluß
- 28: Unterlegscheibe
- 29: Druckfeder
- 30: Dose
- 31: Anschluß

## Patentansprüche

1. Verfahren zum Behandeln des vorderen Nagelrandes von Fingemägeln oder Fußnägeln,
**dadurch gekennzeichnet,**
**daß** eine beheizte Einrichtung am vorderen Nagelrand entlanggeführt und dieser auf eine derartige Temperatur erwärmt wird, daß die Nagelsubstanz etwas schmilzt und der vordere Nagelrand verschweißt und geglättet wird.

2. Verfahren nach dem vorhergehenden Anspruch,
**dadurch gekennzeichnet,**
**daß** die beheizte Einrichtung stabförmig, insbesondere zylinderförmig ausgebildet ist und entweder mit der gekrümmten Mantelfläche (18) oder mit der Stirnfläche (19) am vorderen Nagelrand entlangführbar ist.

3. Verfahren nach einem der vorhergehenden Ansprüche,
**dadurch gekennzeichnet,**
**daß** die beheizte Einrichtung eine schlitzartige Nut (8) zur Aufnahme des vorderen Nagelrandes aufweist.

4. Verfahren nach Anspruch 3,
**dadurch gekennzeichnet,**
**daß** die Nut (8) in der vorderen Stirnseite (7) der beheizbaren Einrichtung ausgebildet ist, wobei diese vordere Stirnseite (7) der beheizten Einrichtung zusammen mit der Nut (8) entweder eben oder konvex oder konkav gekrümmt ist.

5. Verfahren nach Anspruch 2 und 3,
**dadurch gekennzeichnet,**
**daß** die Nut (8) um den Umfang der zylinderförmigen Mantelfläche (18) herum ausgebildet ist.

6. Verfahren nach einem der Ansprüche 1 bis 5,
**dadurch gekennzeichnet,**
**daß** die beheizte Einrichtung als Wärmespeicher mit einer externen Aufheizeinrichtung in der Art eines Ofens ausgebildet ist.

7. Verfahren nach einem der Ansprüche 1 bis 5,
**dadurch gekennzeichnet,**
**daß** die beheizte Einrichtung eine integrierte elektrische Widerstandsheizung aufweist.

8. Verfahren nach Anspruch 7,
**dadurch gekennzeichnet,**
**daß** die Heiztemperatur regelbar ist.

9. Verfahren nach einem der vorhergehenden Ansprüche,
**dadurch gekennzeichnet,**
**daß** die beheizte Einrichtung ein in einem Halter (1) angeordnetes Heizelement (2) aufweist.

10. Verfahren nach Anspruch 9,
**dadurch gekennzeichnet,**
**daß** in dem Heizelement (2) eine separate Heizpatrone (3) oder Heizkissen angeordnet ist.

11. Verfahren nach einem der vorhergehenden Ansprüche,
**gekennzeichnet durch**
eine Zusatzeinrichtung zum Abdecken und/oder Kühlen der nicht zu behandelnden Bereiche des Nagels sowie des Fingers bzw. Zehens.

12. Verfahren nach einem der vorhergehenden Ansprüche,
**dadurch gekennzeichnet,**
**daß** zusätzlich noch eine elektrische Spannung angelegt wird.

## Claims

1. A method for the treatment of the anterior nail edge of finger nails or toe nails,
**characterised in that**
a heated device is passed along the anterior nail edge and this is heated to such a temperature that the nail substance melts slightly and the anterior nail edge is welded and smoothed.

2. The method according to the preceding claim,
**characterised in that**
the heated device is constructed as rod-shaped, especially as cylindrical and can be guided along the anterior nail edge either with the curved shell surface (18) or with the face (19).

3. The method according to one of the preceding claims,
**characterised in that**
the heated device has a slot-like groove (8) to accommodate the anterior nail edge.

4. The method according to claim 3,
**characterised in that**
the groove (8) is constructed in the anterior face (7) of the heatable device wherein this anterior face (7) of the heated device together with the groove (8) is either flat or convexly or concavely curved.

5. The method according to claim 2 or 3,
**characterised in that**
the groove (8) is constructed around the circumference of the cylindrical shell surface (18).

6. The method according to one of claims 1 to 5,
**characterised in that**
the heated device is constructed as a heat storage device with an external heating device in the fashion of an oven.

7. The method according to one of claims 1 to 5,
**characterised in that**
the heated device is constructed as a built-in electrical resistance heater.

8. The method according to claim 7,
**characterised in that**
the heating temperature is controllable.

9. The method according to one of the preceding claims,
**characterised in that**
the heated device has a heating element (2) arranged in a holder (1).

10. The method according to claim 9,
**characterised in that**
a separate heating cartridge (3) or heating pad is arranged in the heating element (2).

11. The method according to one of the preceding claims,
**characterised by**
an auxiliary attachment for covering and/or cooling the areas of the nail and of the finger or the toe not to be treated.

12. The method according to one of the preceding claims,
**characterised in that**
an electrical voltage is additionally applied.

## Revendications

1. Procédé pour le traitement du bord avant des ongles des doigts ou des ongles des pieds,
**caractérisé en ce que**
une installation chauffée est guidée le long du bord avant de l'ongle et que celui-ci est chauffé à une température telle que la substance de l'ongle fonde un peu et que le bord avant de l'ongle soit soudé et lissé.

2. Procédé selon la revendication précédente,
**caractérisé en ce que**
l'installation chauffée est configurée en forme de barre, notamment de forme cylindrique, et peut être guidée longitudinalement soit par sa surface de chemise courbée (18) soit par sa surface frontale (19) sur le bord avant de l'ongle.

3. Procédé selon la revendication précédente,
**caractérisé en ce que**
l'installation chauffée présente une rainure en forme de fente (8) destinée à recevoir le bord avant de l'ongle.

4. Procédé selon la revendication 3,
**caractérisé en ce que**
la rainure (8) est formée dans la face frontale avant (7) de l'installation pouvant être chauffée, la face frontale avant (7) de l'installation chauffée étant avec la rainure (8) soit plane, soit courbée de manière convexe ou concave.

5. Procédé selon les revendications 2 et 3,
**caractérisé en ce que**
la rainure (8) est formée autour de la périphérie de la surface de chemise en forme de cylindre (18).

6. Procédé selon une des revendications 1 à 5,
**caractérisé en ce que**
l'installation chauffée est conçue comme un accumulateur de chaleur avec un dispositif externe de chauffage de type four.

7. Procédé selon une des revendications 1 à 5,
**caractérisé en ce que**
l'installation chauffée présente un chauffage électrique à résistance intégré.

8. Procédé selon la revendication 7,
**caractérisé en ce que**
la température de chauffage est réglable.

9. Procédé selon une des revendications précédentes,
**caractérisé en ce que**
l'installation chauffée présente un élément chauffant (2) disposé dans un support (1).

10. Procédé selon la revendication 9,
**caractérisé en ce que**
une cartouche chauffante séparée (3) ou un coussin chauffant est disposé dans l'élément chauffant (2).

11. Procédé selon une des revendications précédentes,
**caractérisé par**
une installation supplémentaire pour le recouvrement et/ou le refroidissement des zones non à traiter de l'ongle ainsi que du doigt ou de l'orteil.

12. Procédé selon une des revendications précédentes,
**caractérisé en ce que**
en outre une tension électrique est appliquée.
